(19) **Europäisches Patentamt / European Patent Office / Office européen des brevets**

(11) **EP 1 782 801 A1**

(12) # EUROPEAN PATENT APPLICATION
published in accordance with Art. 158(3) EPC

(43) Date of publication:
**09.05.2007 Bulletin 2007/19**

(21) Application number: **05780913.9**

(22) Date of filing: **24.08.2005**

(51) Int Cl.:
*A61K 31/01* (2006.01)   *A61K 9/107* (2006.01)
*A61K 47/32* (2006.01)   *A61K 47/34* (2006.01)
*A61K 47/38* (2006.01)   *A61P 27/02* (2006.01)
*A61P 27/04* (2006.01)   *A61P 27/14* (2006.01)
*A61P 37/08* (2006.01)

(86) International application number:
**PCT/JP2005/015340**

(87) International publication number:
**WO 2006/022291 (02.03.2006 Gazette 2006/09)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **27.08.2004 JP 2004248720**

(71) Applicant: **Senju Pharmaceutical Co., Ltd.
Osaka-shi, Osaka 541-0046 (JP)**

(72) Inventor: **MATSUHISA, Keiichi, Senju
Pharmaceutical Co., Ltd.
Kobe-shi Hyogo 651-2241 (JP)**

(74) Representative: **Behnisch, Werner et al
Reinhard-Skuhra-Weise & Partner GbR
Friedrichstrasse 31
80801 München (DE)**

(54) **EYEWASH LIQUID FOR DRY EYE THERAPY**

(57)   Eye drops for the treatment of dry eye is disclosed. The eye drops is a solution-type, emulsion-type or two separate phase-type eye drops containing squalane and is, among others, an oil-in-water-type emulsion further containing a water-soluble macromolecular compound such as polyvinylpyrrolidone. Also disclosed are use of squalane for the production of eye drops for the treatment of dry eye, and a method for the treatment of dry eye.

**EP 1 782 801 A1**

**Description**

**TECHNICAL FIELD**

The present invention relates to eye drop for the treatment of dry eye, containing squalane as an oily component.

**BACKGROUND ART**

In recent years, incidence of dry eye has been increasing which is associated with contact lens wearing or VDT tasks and the like, due to accumulating factors that contribute to the development of dry eye including growing numbers of contact lens wearers, extending hours spent in artificial, air-conditioned environments and expanding opportunities where to gaze at VDT [video (visual) display terminal] screens as a result of widespread use of TV sets and computers.

In most of dry eye cases, one of the layers which make up the tear film, i.e., the oil layer, water layer or mucous layer, fails, which then causes corneal/conjunctival disorders. Among others, failure of the mucous layer causes severe corneal disorders, which likely leads not only to disorders of corneal epithelium or erosion of corneal epithelium due to the disorder in corneal epithelial cells, but also to corneal ulcer (e.g., ulcer of the corneal stroma and the like) and ophthalmic infections, in some cases of which corneal implantation becomes necessary.

At present, the most popular treatment of dry eye is topical application of an artificial tear containing a viscoelastic compound as a substitute for mucin, such as methylcellulose, chondroitin sulfate and hyaluronic acid. However, these compounds differ from mucin both physically and physiologically, and its therapeutic efficacy, therefore, is limited. On the other hand, topical application of an oily component alone to the eye, for the purpose of supplementing it, would cause blurred vision and thereby result in poor compliance.

The prior art relating to the treatment of dry eye includes, in addition to the above, what are described, e.g., in the following laid open patent applications.

Patent Document 1 discloses eye drops containing a slight amount of oil for dry eye treatment consisting of artificial tear-based eye drops to which is added a slight amount of oil, e.g., castor oil or liquid paraffin.

Patent Document 2 discloses an artificial tear for ophthalmic use which, on the basis artificial tear for ophthalmic use containing physiological metal ions, esp. alkali metal ions, further contains supplementary alkaline earth metal ions, and is adjusted to have osmotic pressure of 220-295 $mOsm/kgH_2O$ and pH of 6-8, and also an artificial tear for ophthalmic use of this type but further containing dispersed castor oil.

Patent Document 3 discloses a heat-gelating artificial tear containing (a) methylcellulose and (b) at least one compound consisting of polyethylene glycol, citric acid and its pharmaceutically acceptable salt, and a heat-gelating artificial tear further containing chondroitin sulfate or its pharmaceutically acceptable salt.

Patent Document 4 discloses a heat-gelating artificial tear containing methylcellulose and taurine.

Patent Document 5 discloses an artificial tear film on the surface of the eyeball containing an aqueous layer which is covered with an oil film.

Further, Patent Document 6 discloses a method for the treatment of dry eye, consisting of applying a gelating oil to the eye, preferably in the form of metastable oil-in-water type emulsion, at a dose of not exceeding 100 μL.

However, none of these conventionally known methods for treatment is satisfactory, and thus, there are potential needs for new eye drops for the treatment of dry eye.

[Patent Document 1] Japanese Patent Application Publication H10-218760
[Patent Document 2] Japanese Patent Application Publication 2000-159659
[Patent Document 3] Japanese Patent Application Publication 2003-95924
[Patent Document 4] Japanese Patent Application Publication 2003-267892
[Patent Document 5] Japanese Patent Application Publication H4-279525
[Patent Document 6] Japanese Patent Application Publication H7-2647

**DISCLOSURE OF INVENTION**

Against the above background, the objective of the present invention is to provide eye drops for the treatment of dry eye which is highly effective in suppressing evaporation of water from the eye.

The present inventors, as a result of studies made to achieve the above objective, found that, in eye drops containing an oily ingredient in addition to aqueous ingredients, use of squalane as the oily ingredient brings about enhancement of the eye drops' suppressive effect on water evaporation. Further, through additional finding that emulsion-type eye drops which is prepared by emulsifying squalane with a water soluble macromolecular compound such as polyvinylalcohol and the like has greater suppressive effect on water evaporation. The present invention was completed on the basis of these findings.

Thus, the present invention provides what follows:

(1) Eye drops for the treatment of dry eye comprising squalane.
(2) The eye drops as defined in (1) above further comprising a water-soluble macromolecular compound.
(3) The eye drops as defined in (1) or (2) above which is an oil-in-water emulsion.
(4) The eye drops as defined in (2) or (3) above, wherein the water-soluble macromolecular compound is selected from the group consisting of polyvinylalcohol, polyvinylpyrrolidone, hydroxypropylmethylcellulose, methylcellulose, polyethylene glycol, hydroxyethylcellulose and carboxyvinylpolymer.
(5) The eye drops as defined in one of (2) to (4) above which contain polyvinylalcohol as the water-soluble macromolecular compound.
(6) The eye drops as defined in one of (1) to (5) above, wherein the concentration of squalane is not less than 0.01 W/V% and not more than 20 W/V%.
(7) The eye drops as defined in one of (2) to (6) above, wherein the concentration of the water-soluble macromolecular compound is not less than 0.01 W/V% and not more than 20 W/V%.
(8) The eye drops as defined in one of (1) to (7) above, wherein the pH thereof is 5.5-8.0 and the relative osmotic pressure thereof (the ratio of its osmotic pressure to that of physiological saline) is 0.85-1.55.
(9) A method for preparation of eye drops for the treatment of dry eye comprising emulsifying squalane with a water-soluble macromolecular compound.
(10) The eye drops as defined in one of (1) to (9) above, wherein said dry eye is dry eye accompanying lacrimal fluid reduction, tear deficiency, xerosis of the eye, Sjogren's syndrome, keratoconjunctivitis sicca, Stevens-Johnson syndrome, pemphigoid of the eye, marginal blepharitis, failure in eyelids closure, or sensory nerve numbness, dry eye accompanying allergic conjunctivitis, dry eye after viral conjunctivitis, dry eye after cornea surgery including laser in situ keratomileusis (LASIK), dry eye after cataract surgery, dry eye associated with contact lens wearing, or dry eye associated with VDT tasks.
(11) Use of squalane for the production of eye drops for the treatment of dry eye.
(12) The use as defined in (11) above, wherein said dry eye is dry eye accompanying lacrimal fluid reduction, tear deficiency, xerosis of the eye, Sjogren's syndrome, keratoconjunctivitis sicca, Stevens-Johnson syndrome, pemphigoid of the eye, marginal blepharitis, failure in eyelids closure, or sensory nerve numbness, dry eye accompanying allergic conjunctivitis, dry eye after viral conjunctivitis, dry eye after cornea surgery including laser in situ keratomileusis (LASIK), dry eye after cataract surgery, dry eye associated with contact lens wearing, or dry eye associated with VDT tasks.
(13) A method for the treatment of dry eye, wherein the method comprises applying eye drops comprising squalane at a therapeutically effective concentration to the eyes of a human patient with dry eye.
(14) The method for the treatment as defined in (13) above, wherein the eye drops further comprises a water-soluble macromolecular compound.
(15) The method for the treatment as defined in (13) or (14) above, wherein the eye drops is an oil-in-water type emulsion.
(16) The method for the treatment as defined in (14) or (15) above, wherein the water-soluble macromolecular compound is selected from the group consisting of polyvinylalcohol, polyvinylpyrrolidone, hydroxypropylmethylcellulose, methylcellulose, polyethylene glycol, hydroxyethylcellulose and carboxyvinylpolymer.
(17) The method for the treatment as defined in one of (14) to (16) above, wherein the eye drops contains polyvinylalcohol as a water-soluble macromolecular compound.
(18) The method for the treatment as defined in one of (13) to (17) above, wherein the concentration of squalane is not less than 0.01 W/V% and not more than 20 W/V%.
(19) The method for the treatment as defined in one of (14) to (18) above, wherein the concentration of the water-soluble macromolecular compound is not less than 0.01 W/V% and not more than 20 W/V%.
(20) The method for the treatment as defined in one of (13) to (19) above, wherein the pH of the eye drops is 5.5-8.0 and the relative osmotic pressure thereof (the ratio of its osmotic pressure to that of physiological saline) is 0.85-1.55.

[EFFECT OF THE INVENTION]

The present invention provides eye drops for the treatment of dry eye comprising squalane and having enhanced suppressive effect on water evaporation, and also a method for the treatment of dry eye, based on suppression of water evaporation through application of such eye drops to patients' eyes.

[BEST MODE FOR CARRYING OUT THE INVENTION]

Squalane, which is used in the eye drops of the present invention, is a saturated hydrocarbon which is obtained by reduction of squalene, which is an unsaturated hydrocarbon occurring in liver oil of fish living in deep sea, esp. sharks, and in vegetable oils such as olive oil, rice oil, wheat gem oil, sesame oil, and cotton seed oil. Squalane used in the present invention may be either one obtained by reduction of naturally occurring squalene or synthetic squalane as is obtainable by synthesis starting with, e.g., geranylacetone and an acetylene compound as raw materials.

The concentration of squalane contained in the eye drops for the treatment of dry eye of the present invention is generally not less than 0.01 W/V%, preferably not less than 0.1 W/V%, more preferably not less than 0.5 W/V%, and generally not more than 20 W/V%, preferably not more than 10 W/V%, and more preferably not more than 5 W/V%.

Examples of the water-soluble macromolecular compound employed in the eye drops for the treatment of dry eye of the present invention include polyvinylalcohol (PVA), polyvinylpyrrolidone (PVP), hydroxypropylmethylcellulose (HPMC), methylcellulose (MC), polyethylene glycol (PEG), hydroxyethylcellulose (HEC), and carboxyvinylpolymer and the like.

Though it depends on a compound employed and the concentration of squalane, the concentration of the water-soluble macromolecular compound contained in the eye drops for the treatment of dry eye of the present invention is generally not less than 0.01 W/V%, preferably not less than 0.1 W/V%, more preferably not less than 0.5 W/V%, and generally not more than 20 W/V%, preferably not more than 10 W/V%, and more preferably not more than 5 W/V%. In the case of polyvinylalcohol, its preferable concentration ranges somewhere from 0.1 W/V% to 5 W/V%.

The eye drops for the treatment of dry eye of the present invention may further contain one or more other ingredients which can be contained in artificial tears, i.e., aminoethylsulfonic acid, sodium chondroitin sulfate, potassium L-aspartate, magnesium L-aspartate, potassium magnesium L-aspartate (equimolar mixture), sodium hydrogen carbonate, sodium carbonate, potassium chloride, calcium chloride, sodium chloride, sodium hydrogen phosphate, sodium dihydrogen phosphate, potassium dihydrogen phosphate, exsiccated sodium carbonate, magnesium sulfate, polyvinylalcohol, polyvinylpyrrolidone, hydroxyethylcellulose, hydroxypropylmethylcellulose, glucose, and methylcellulose.

The eye drops for the treatment of dry eye of the present invention is useful for the treatment of dry eye accompanying lacrimal fluid reduction, tear deficiency, xerosis of the eye, Sjogren's syndrome, keratoconjunctivitis sicca, Stevens-Johnson syndrome, pemphigoid of the eye, marginal blepharitis, failure in eyelids closure, or sensory nerve numbness, dry eye associated with allergic conjunctivitis, dry eye after viral conjunctivitis, dry eye after cornea surgery including laser in situ keratomileusis (LASIK), dry eye after cataract surgery, dry eye associated with contact lens wearing, or dry eye associated with VDT tasks.

The eye drops for the treatment of dry eye of the present invention preferably contains one or more conventional additives as desired insofar as they do not contradict the objective of the present invention. As such additives, buffering agents, isotonizers, preservatives, solubilizing agent, pH adjusting agents, thickeners, chelating agents, and the like, for example, are employed

As buffering agents, among the above additives, phosphate buffer, borate buffer, citrate buffer, tartrate buffer, acetate buffer, amino acids, for example, are employed. As isotonizers, sugars such as sorbitol, glucose, mannitol, polyols such as glycerol, polyethylene glycol, propylene glycol, and salts such as sodium chloride, and the like, for example, are used. As preservatives, benzalkonium chloride, benzethonium chloride, chlorhexidine gluconate, p-hydroxybenzoates such as methyl p-hydroxybenzoate, ethyl p-hydroxybenzoate, benzyl alcohol, phenethyl alcohol, sorbic acid or its salts, thimerosal, chlorobutanol, and the like, for example, are used. As solubilizing agents, cyclodextrins and their derivatives, water-soluble macromolecular compounds such as polyvinylpyrrolidone, surfactants such as polysorbate 80 (trade name: Tween 80), and the like, for example, are used. As pH adjusting agents, hydrochloric acid, acetic acid, phosphoric acid, sodium hydroxide, potassium hydroxide, ammonium hydroxide, and the like, for example, are used. As thickeners, hydroxyethylcellulose, hydroxypropylcellulose, methylcellulose, hydroxypropylmethylcellulose, carboxymethylcellulose and its salts, and the like, for example, are used. As chelating agent, sodium edetate, sodium citrate, condensed sodium phosphate, and the like, for example, are used.

The eye drops for the treatment of dry eye of the present invention may be in the form of oil-in-water (O/W type) emulsion, micro emulsion, and the like, or in a form consisting of two separate phases, i.e., an aqueous phase and an oil phase (referred to in the present specification as a "two separate-phase type"). In the case of a two separate-phase type, the eye drops is shaken before use. In the case where it is in the form of an emulsion or a micro emulsion, the average size of its oil droplets is preferably 5-0.0001 $\mu$m, more preferably 1-0.001 $\mu$m, and particularly preferably 1-0.01 $\mu$m. The average size can be determined on a particle size analyzer.

The eye drops for the treatment of dry eye of the present invention has its pH at about 5.5-8, relative osmotic pressure at 0.85-1.55 (the ratio of its osmotic pressure to that of physiological saline).

In the case where the eye drops for the treatment of dry eye of the present invention is to be provided in the form of emulsion, a technique known in the art for preparing emulsion is employed. For example, squalane, a water-soluble macromolecular compound such as PVA and, as needed, some of the above-mentioned additives may be added to water, and the mixture, after being adjusted to pH 5.5-8 with a pH adjusting agent, stirred and mixed to form an emulsion. For achieving homogeneous emulsification, a means known in the art may be employed, such as a homomixer, homogenizer, microfluidizer, high-pressure homogenizer, and the like.

The eye drops for the treatment of dry eye of the present invention is preferably administered about 1-8 times a day, one to a few drops at a time, depending on the symptom.

One or more additional ingredients for treating dry eye may further be added as desired to the eye drops for the treatment of dry eye of the present invention, insofar as they do not contradict the objective of the present invention, e.g., a viscoelastic compound such as hyaluronic acid, and the like.

[EXAMPLES]

The present invention will be further described with reference to examples and the efficacy of the present invention will be demonstrated with reference to test examples. However, they are just examples, and it is not intended to limit the scope of the present invention with those examples.

[Test Example 1] Selection of oil

Castor oil (Japanese Pharmacopoeia), liquid paraffin (Japanese Pharmacopoeia), cotton seed oil (Nacalai Tesque, Inc.), olive oil (Nacalai Tesque, Inc.), squalane (Nacalai Tesque, Inc.) were chosen as candidate oils. According to the compositions described in Table 1, an oil, PVA and water (0.5:1:100) were mixed and a 4-ml aliquot of each of the mixture liquid prepared by stirring on a homomixer was dispensed in a petri dish, and stored at 37 °C and 50 % RH. The weight of the aliquots was measured every one hour up to 5 hours to determine the weight of water that had evaporated.

[Table 1]

Table 1. Mixtures Liquid Tested

|  | Water | [1] | [2] | [3] | [4] | [5] |
|---|---|---|---|---|---|---|
| Water | 100 | 100 | 100 | 100 | 100 | 100 |
| PVA | 1 | 1 | 1 | 1 | 1 | 1 |
| Castor oil | - | 0.5 | - | - | - | - |
| Liquid paraffin | - | - | 0.5 | - | - | - |
| Cotton seed oil | - | - | - | 0.5 | - | - |
| Olive oil | - | - | - | - | 0.5 | - |
| Squalane | - | - | - | - | - | 0.5 |

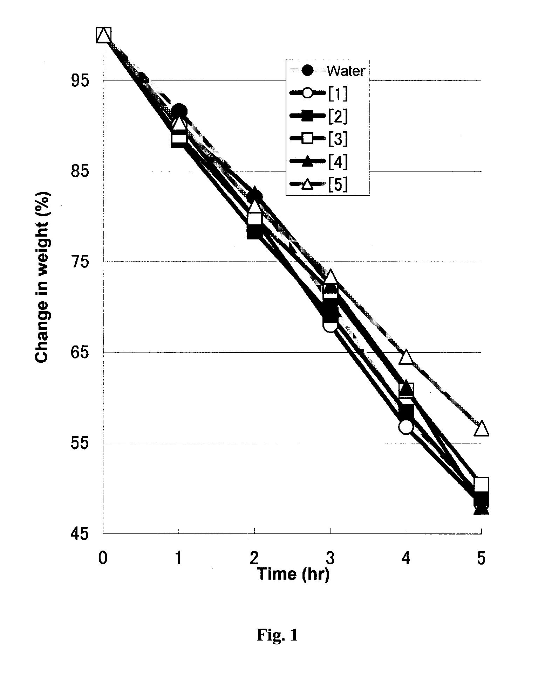

The results are shown in Fig. 1. Evaporation of water was found the most suppressed in the mixture liquid containing squalane (Mixture solution [5], indicated by open triangles).

[Test Example 2] Suppression of Water Evaporation from the Surface of Rabbit Cornea/Conjunctiva

1. Test preparations

As test preparations, the eye drops indicated as formula A in Table 2 containing squalane as the oily ingredient, and base of it were used. The eye drops of formula A employed had been prepared by rough emulsification on a homomixer, followed by atomization on a microfluidizer (M-110 EH, manufactured by MIZUHO INDUSTRIAL CO., LTD.) under the conditions of 1500 BAR and, 10 pass.

[Table 2]

Table 2. Test Preparations

|  | Formula A | Base of Formula A |
|---|---|---|
| Squalane | 0.7 g | - |
| Gosenol EG-05* | 0.15 g | 0.15 g |
| Conc. glycerol | 1.0 g | 1.0 g |
| Sodium chloride | 0.55 g | 0.55 g |
| Potassium chloride | 0.16 g | 0.16 g |
| Sodium carbonate | 0.06 g | 0.06 g |
| Sodium hydrogen phosphate | 0.18 g | 0.18 g |
| Benzalkonium chloride | 0.005 g | 0.005 g |
| Hydrochloric acid | q.s. | q.s. |
| Purified water | q.s. | q.s. |
| pH | 7.0 | 7.0 |
| * Polyvinylalcohol (The Nippon Synthetic Chemical Industry Co., Ltd.) | | |

2. Animals used

Male, nine week-old Japanese albino rabbits (purchased from KITAYAMA LABES Co., Ltd.) were used in the test. They were fed with solid type Labo R Stock (Nosan Corporation), which was given 100 g a day. They were kept, one animal in a cage, until the end of the test at $23 \pm 3$ °C, $55 \pm 10$ % RH, in a breeding room set at 12-hour lighting (on at 8:00, off at 20:00), with free access to water from an automatic water supply system..

3. Test method

1) Selection of the animals and their assignment to the groups

Sixteen animals were chosen which showed no abnormal signs either in their general conditions or in the eyes, and they were assigned to one of four groups I-IV, four animals each.

2) Administration

Administration of the test preparations was done using a micropipetter. Formula A eye drops was topically applied to one of the eyes, and the base of formula A to the other, 100 µL per eye.

3) Test procedures

The faces of the rabbits were shaved by the day on which topical application to the eyes was made. On the day when the topical application to the eyes was made, the animals were anesthetized with intramuscular injection of ketaral (ketaral hydrochloride, Sankyo Co., Ltd.) and seractal (xylazine hydrochloride, Bayer Medical Ltd.) (2.4:1, total amount; 0.85 mL/kg). Twenty % N-acetylcysteine solution, 50 µL at a time, was instilled to the eyes three times, i.e., 20, 15 and 10 minutes to remove the mucin layer before the application of either of the test preparations. Then, 100 µL of one or the other of the test preparations was topically applied once to the eyes of the animals of groups I-III. In order to measure the extent of water evaporation from the surface of the cornea/conjunctiva, the eyes were kept open with surgical tapes applied to the upper and lower eyelids, and a chamber (*) was mounted on the eyelids so as to cover each of the eyes with it, the humidity inside of which was measured every minute. The measurement of humidity was performed immediately after the topical application of one of the test preparations (0 min) and up to 10 minutes after the application for the group I animals, and from 10 to 20 minutes after the application of the test preparations for the group II animals, and from 20 to 30 minutes after the application of the test preparations for the group III animals, respectively. The eyelids of the animals were kept closed with surgical tapes until the measurement of humidity was started. As controls, the IV animals received no test preparations after the removal of the mucin layer, and their eyelids were kept closed with surgical tapes applied on them, and the humidity inside of the chambers mounted on them was measured from 0 to 10 minutes after the removal of the mucin layer. Since the eyelids of the group IV animals were kept closed, the evaporation of water measured with the group of animals represented the evaporation from the skin of the eyelids, and is thought to be constant through the time during which the experiment was done, regardless of the time frame within which the measurement was performed. Therefore, this result of the measurement was commonly used in the calculation of the evaporation coefficient from the surface of the cornea/conjunctiva within each of the time frame in groups I-III, as described later. The conditions of the measurement are listed in Table 3.

Table 3. Humidity measurement

| Group | Test preparation | Time for measurement | Eyelids during humidity measurement |
|---|---|---|---|
| I | applied | 0-10min | Open |
| II | applied | 10-20 min | Open |
| III | applied | 20-30 min | Open |
| IV | not applied | 0-10 min | Closed |

**[0041]** * Chamber: A chamber (volume: 70 mL) was prepared by inserting a humidity sensor through a hole made in the bottom of a 50 mL centrifuge tube (polypropylene tube, manufactured by Iwaki Glass Co., Ltd.), and connecting a swimming goggle at the upper part of the centrifuge tube. Silicone bond was applied to the site of connection to keep its air□tightness. As a humidity meter, a digital thermohygrometer meter (SK-110 TRH, manufactured by Sato Keiryoki Mfg. Co., Ltd.) was used.

4) Calculation of the evaporation coefficient

According to an equation described in a literature (Masakazu Yamada and Kazuo Tsuboi, "Measurement of Tear Evaporation from Ocular Surface", Acta□Soc□Ophthalmol Jpn 94(11): 1061-1067, 1990), the evaporation coefficient K was calculated based on the measurement of the humidity for 5 minutes after the start of the measurement, during which a steady increase in humidity was measurable within the closed space (chamber). Thus, the evaporation coefficient (K) from the cornea/conjunctiva is defined by the following formula:

[Math 1]

$$K = (\text{coefficient } k' \text{ with open eyelids}) - (\text{coefficient } k' \text{ with closed eyelids})$$

In the formula, coefficients k' are the values derived from the following formulas, under the condition with open or closed eyelids, respectively, wherein $H_0$ (%) is the initial humidity in the chamber, and H (%) the humidity t seconds after the chamber was mounted.

[Math 2]

$$100 - H = (100 - H_0) \exp(-k't)$$

Based on the humidity measured, k' can be determined, for example, from the slope of a semilogarithmic graph produced by plotting "100-H" in logarithmic scale relative to time t.

4. Test results

The results are shown in Table 2. In each of the time frames in which the measurement done, i.e., from 0 minute to 10 minutes (group 1), from 10 minutes to 20 minutes (group II), and from 20 minutes to 30 minutes (group III) after the topical application, the change in humidity in the chamber attached to the eyes which had received topical application of formula A was kept lower compared to that with the eyes which had received topical application of the base. This indicates that water evaporation was lower from the eyes which had received eye drops containing squalane. Meanwhile, in group IV, in which measurement was made without application of a test preparation and with eyelids closed, the change in humidity from 0 minute to 10 minutes was the lowest. The measured humidity in this group reflected water evaporation from the surface of the eyelid skin, and the difference between it and the measured humidity with the eyelids kept open corresponds to the amount of the water which evaporated from the cornea/conjunctiva (mean value from four animals for groups I to III, mean value from 8 animals for group IV).

From the changes in humidity within the chambers in groups I to III, from 0 minute to 5 minutes, from 10 minutes to 15 minutes, and from 20 minutes to 25 minutes, respectively, after the topical application of one of the test preparations, and the changes in humidity within the chamber in group IV, the evaporation coefficients K were derived, for the eyes to which formula A eye drops had been topically applied, as well as for the eyes to which the base had been topically applied. The results are shown in Table 4.

Table 4. Evaporation coefficient K (x $10^{-4}$/sec) after application of squalane-containing eye drops (formula A) or its base.

| Time for measurement<br>Application | 0-5 min | 10-15 min | 20-25 min |
|---|---|---|---|
| Formula A | 7.4±6.0 | 8.5±6.5 | 9.3±4.4 |
| Base | 12.3±4.8 | 12.2±3.3 | 11.5±2.8 |
| □Values represent mean ± standard deviation (n = 4). | | | |

As seen in Table 4, the evaporation coefficient K in the formula A-applied eyes was smaller than that in the base-applied eyes. Also from this comparison in evaporation coefficient, it is evident that application of squalane-containing eye drops suppresses evaporation of water from the cornea/conjunctiva.

Example 1

Eye drops

| | |
|---|---|
| Squalane | 0.7 W/V% |
| Gosenol EG-05 | 0.15 |
| Conc. glycerol | 1.0 |
| Sodium chloride | 0.55 |
| Potassium chloride | 0.16 |
| Sodium carbonate | 0.06 |
| Sodium hydrogen phosphate | 0.18 |
| Benzalkonium chloride | 0.005 |
| Hydrochloric acid | q.s. |
| Purified water | q.s. |
| Total volume 100 mL (pH 7.0) | |

The above ingredients were mixed in a conventional manner to form emulsion-type eye drops.

Example 2

Eye drops

| | |
|---|---|
| Squalane | 1.0 W/V% |
| Gosenol EG-05 | 0.2 |
| Conc. glycerol | 1.0 |
| Sodium chloride | 0.55 |
| Potassium chloride | 0.16 |

(continued)

| | |
|---|---|
| Squalane | 1.0 W/V% |
| Sodium carbonate | 0.06 |
| Sodium hydrogen phosphate | 0.18 |
| Benzalkonium chloride | 0.005 |
| Hydrochloric acid | q.s. |
| Purified water | q.s. |
| Total volume 100 mL (pH 7.0) | |

The above ingredient were mixed in a conventional manner and then miniaturized on a microfluidizer to form emulsion-type eye drops.

Example 3

| Eye drops | |
|---|---|
| Squalane | 5.0 W/V% |
| Gosenol EG-05 | 1.0 |
| Conc. glycerol | 1.0 |
| Sodium chloride | 0.55 |
| Potassium chloride | 0.16 |
| Sodium carbonate | 0.06 |
| Sodium hydrogen phosphate | 0.18 |
| Benzalkonium chloride | 0.005 |
| Hydrochloric acid | q.s. |
| Purified water | q.s. |
| Total volume 100 mL (pH 7.0) | |

The above ingredients were mixed in a conventional manner to form emulsion-type eye drops.

Example 4

| Eye drops | |
|---|---|
| Squalane | 0.7 W/V% |
| Gosenol EG-05 | 1.0 |
| Conc. glycerol | 1.0 |
| Sodium chloride | 0.55 |
| Potassium chloride | 0.16 |
| Sodium carbonate | 0.06 |
| Sodium hydrogen phosphate | 0.18 |
| Chlorhexidine gluconate | 0.0025 |
| Hydrochloric acid | q.s. |
| Purified water | q.s. |
| Total volume 100 mL (pH 7.0) | |

The above ingredients were mixed in a conventional manner to form emulsion-type eye drops.

Example 5

<div align="center">Eye drops</div>

| | |
|---|---|
| Squalane | 0.7 W/V% |
| Hydroxypropylmethylcellulose | 0.1 |
| Boric acid | 1.2 |
| Sodium chloride | 0.55 |
| Potassium chloride | 0.16 |
| Sodium carbonate | 0.06 |
| Sodium hydrogen phosphate | 0.18 |
| Chlorhexidine gluconate | 0.0025 |
| Hydrochloric acid | q.s. |
| Purified water | q.s. |
| Total volume 100 mL (pH 7.0) | |

The above ingredients were mixed in a conventional manner to form emulsion-type eye drops.

Example 6 Eye drops

| | |
|---|---|
| Squalane | 0.7 W/V% |
| Gosenol EG-05 | 0.15 |
| Conc. glycerol | 1.0 |
| Sodium chloride | 0.55 |
| Potassium chloride | 0.16 |
| Sodium carbonate | 0.06 |
| Sodium hydrogen phosphate | 0.18 |
| Benzalkonium chloride | 0.005 |
| Hydrochloric acid | q.s. |
| Purified water | q.s. |
| Total volume 100 mL (pH 7.0) | |

Using the above ingredients, eye drops consisting of separate oil and water phases was prepared in a conventional manner. It is shaken before use.

[INDUCTRIAL APPLICABILITY]

The eye drops for the treatment of dry eye of the present invention, which has an oil phase and an aqueous phase, is useful as a prophylactic and therapeutic drug for dry eye attributable to any of a variety of causes, such as dry eye accompanying lacrimal fluid reduction, tear deficiency, xerosis of the eye, Sjogren's syndrome, keratoconjunctivitis sicca, Stevens-Johnson syndrome, pemphigoid of the eye, marginal blepharitis, failure in eyelids closure, or sensory nerve numbness, dry eye accompanying allergic conjunctivitis, dry eye after viral conjunctivitis, dry eye after cornea surgery including laser in situ keratomileusis (LASIK), dry eye after cataract surgery, dry eye associated with contact lens wearing, or dry eye associated with VDT tasks.

[BRIEF DESCRIPTIOHN OF THE DRAWINGS]

Fig. 1 is a graph illustrating the change in weight of each of the mixture liquids of test example 1 (one sample each) on a petri dish.

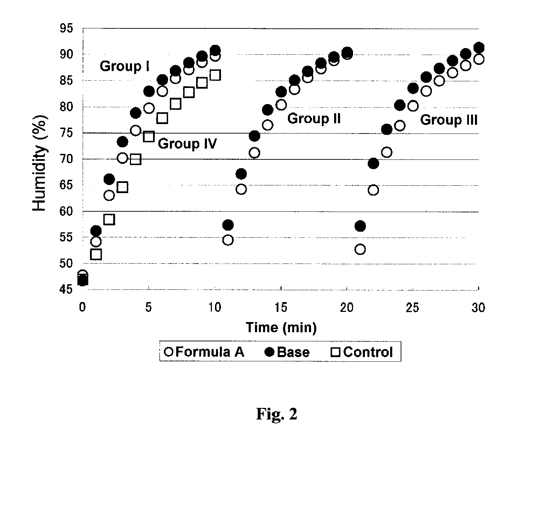

Fig. 2 is a graph illustrating the changes in humidity within the chambers mounted on the eyes of the animals of each group in test example 2.

**Claims**

1. Eye drops for the treatment of dry eye comprising squalane.

2. The eye drops of claim 1 further comprising a water-soluble macromolecular compound.

3. The eye drops of claim 1 or 2 which is an oil-in-water emulsion.

4. The eye drops of claim 2 or 3, wherein the water-soluble macromolecular compound is selected from the group consisting of polyvinylalcohol, polyvinylpyrrolidone, hydroxypropylmethylcellulose, methylcellulose, polyethylene glycol, hydroxyethylcellulose and carboxyvinylpolymer.

5. The eye drops of one of claims 2 to 4 which contain polyvinylalcohol as a water-soluble macromolecular compound.

6. The eye drops of one of claims 1 to 5, wherein the concentration of squalane is not less than 0.01 W/V% and not more than 20 W/V%.

7. The eye drops of one of claims 2 to 6, wherein the concentration of the water-soluble macromolecular compound is not less than 0.01 W/V% and not more than 20 W/V%.

8. The eye drops of one of claims 1 to 7, wherein the pH thereof is 5.5-8.0 and the relative osmotic pressure thereof (the ratio of its osmotic pressure to that of physiological saline) is 0.85-1.55.

9. A method for preparation of eye drops for the treatment of dry eye comprising emulsifying squalane with a water-soluble macromolecular compound.

10. The eye drops of one of claims 1 to 9, wherein said dry eye is dry eye accompanying lacrimal fluid reduction, tear deficiency, xerosis of the eye, Sjogren's syndrome, keratoconjunctivitis sicca, Stevens-Johnson syndrome, pemphigoid of the eye, marginal blepharitis, failure in eyelids closure, or sensory nerve numbness, dry eye accompanying allergic conjunctivitis, dry eye after viral conjunctivitis, dry eye after cornea surgery including laser in situ keratomileusis (LASIK), dry eye after cataract surgery, dry eye associated with contact lens wearing, or dry eye associated with VDT tasks.

11. Use of squalane for the production of eye drops for the treatment of dry eye.

12. The use of claim 11, wherein said dry eye is dry eye accompanying lacrimal fluid reduction, tear deficiency, xerosis of the eye, Sjogren's syndrome, keratoconjunctivitis sicca, Stevens-Johnson syndrome, pemphigoid of the eye, marginal blepharitis, failure in eyelids closure, or sensory nerve numbness, dry eye accompanying allergic conjunctivitis, dry eye after viral conjunctivitis, dry eye after cornea surgery including laser in situ keratomileusis (LASIK), dry eye after cataract surgery, dry eye associated with contact lens wearing, or dry eye associated with VDT tasks.

13. A method for the treatment of dry eye, wherein the method comprises applying eye drops comprising squalane at a therapeutically effective concentration to the eyes of a human patient with dry eye.

14. The method for the treatment of claim 13, wherein the eye drops further comprises a water-soluble macromolecular compound.

15. The method for the treatment of claim 13 or 14, wherein the eye drops is an oil-in-water type emulsion.

16. The method for the treatment of claim 14 or 15, wherein the water-soluble macromolecular compound is selected from the group consisting of polyvinylalcohol, polyvinylpyrrolidone, hydroxypropylmethylcellulose, methylcellulose, polyethylene glycol, hydroxyethylcellulose and carboxyvinylpolymer.

17. The method for the treatment of one of claims 14 to 16, wherein the eye drops contains polyvinylalcohol as a water-soluble macromolecular compound.

18. The method for the treatment of one of claims 13 to 17, wherein the concentration of squalane is not less than 0.01 W/V% and not more than 20 W/V%.

**19.** The method for the treatment of one of claims 14 to 18, wherein the concentration of the water-soluble macromolecular compound is not less than 0.01 W/V% and not more than 20 W/V%.

**20.** The method for the treatment of one of claims 13 to 19, wherein the pH of the eye drops is 5.5-8.0 and the relative osmotic pressure thereof (the ratio of its osmotic pressure to that of physiological saline) is 0.85-1.55.

**Fig. 1**

**Fig. 2**

<div align="center">

**INTERNATIONAL SEARCH REPORT**

</div>

| | International application No. |
|---|---|
| | PCT/JP2005/015340 |

| A. CLASSIFICATION OF SUBJECT MATTER |
|---|
| Int.Cl$^7$ A61K31/01, 9/107, 47/32, 47/34, 47/38, A61P27/02, 27/04, 27/14, 37/08 |

According to International Patent Classification (IPC) or to both national classification and IPC

| B. FIELDS SEARCHED |
|---|
| Minimum documentation searched (classification system followed by classification symbols) |
| Int.Cl$^7$ A61K31/01, 9/107, 47/32, 47/34, 47/38, A61P27/02, 27/04, 27/14, 37/08 |

Documentation searched other than minimum documentation to the extent that such documents are included in the fields searched

| Jitsuyo Shinan Koho | 1922-1996 | Jitsuyo Shinan Toroku Koho | 1996-2005 |
|---|---|---|---|
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAOLD(STN), CAplus(STN), REGISTRY(STN), JSTPlus(JOIS)

| C. DOCUMENTS CONSIDERED TO BE RELEVANT | | |
|---|---|---|
| Category* | Citation of document, with indication, where appropriate, of the relevant passages | Relevant to claim No. |
| X | JP 10-218760 A (Yugen Kaisha Nobel Igaku Kenkyusho), 18 August, 1998 (18.08.98), (Family: none) | 1-12 |
| X | JP 4-279525 A (Ocular Research of Boston, Inc.), 05 October, 1992 (05.10.92), & US 5278151 A & US 5294607 A | 1-12 |
| X | JP 7-2647 A (Ocular Research of Boston, Inc.), 06 January, 1995 (06.01.95), & US 5371108 A & EP 535545 A1 | 1-12 |

☐ Further documents are listed in the continuation of Box C.  ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 09 September, 2005 (09.09.05) | 27 September, 2005 (27.09.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

| International application No. |
| --- |
| PCT/JP2005/015340 |

---

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 13-20
   because they relate to subject matter not required to be searched by this Authority, namely:
   The inventions as set forth in claims 13 to 20 pertain to methods for treatment of the human body by therapy.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐ The additional search fees were accompanied by the applicant's protest.

                         ☐ No protest accompanied the payment of additional search fees.

Form PCT/ISA/210 (continuation of first sheet (2)) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H10218760 B
- JP 2000159659 A
- JP 2003095924 A
- JP 2003267892 A
- JP H4279525 B
- JP H72647 A

**Non-patent literature cited in the description**

- **MASAKAZU YAMADA ; KAZUO TSUBOI.** Measurement of Tear Evaporation from Ocular Surface. *Acta Soc Ophthalmol Jpn,* 1990, vol. 94 (11), 1061-1067